# EUROPÄISCHE PATENTANMELDUNG

(11) **EP 1 598 035 A1**
(43) Veröffentlichungstag der Anmeldung: **23.11.2005**
(21) Anmeldenummer: 05007900.3
(22) Anmeldetag: 11.04.2005
(51) Int. Cl.: A61F 2/52

(54) **Brustprothese**

(30) Priorität: 17.05.2004 DE 202004007921 U
(71) Anmelder: Amena Medizin-Orthopädie-Technik GmbH, 83064 Raubling (DE)
(72) Erfinder: Wild, Helmut, 83022 Rosenheim (DE)
(74) Vertreter: Laufhütte, Dieter

(57) **Zusammenfassung**

Die Erfindung betrifft eine Brustprothese im wesentlichen bestehend aus in Kunststofffolien eingeschweißten, der Brustform nachgebildeten Körpern aus einer Silikon-Kautschuk-Masse oder einem anderen weich-elastischen Material. Erfindungsgemäß besteht zumindest ein Teil der Kunststofffolien aus gasdichtem Material.

## Beschreibung

Die Erfindung betrifft eine Brustprothese nach dem Oberbegriff des Anspruchs 1.

Beispielsweise aus der DE 27 01 627 A1 ist ein Verfahren zur Herstellung von Brustprothesen aus in Kunststofffolien eingeschweißten, der Brustform nachgebildeten schalenförmigen Körpern aus einer additionsvemetzenden Zweikomponenten-Silikonkautschuk-Masse bekannt. Die mit diesem Verfahren hergestellten Prothesen sind in ihrem Aussehen und in ihrem Verhalten wegen der elastischen Weichheit, der Beweglichkeit, der Konsistenz, sowie des Gewichts des verwendeten Materials der natürlichen Brust in nahezu idealer Weise nachgebildet.

Die Brustprothesen werden möglichst unverrutschbar an der Brust der Trägerin befestigt. Hierzu ist es beispielsweise aus der EP 392960 A1 bekannt, Brustprothesen der eingangs angegebenen Art innerhalb eines umlaufenden lippenförmigen Randes auf ihrer Rückseite mit einer umlaufenden, durch einen Absatz gebildeten Schulter zu versehen, auf der Haftstreifen oder Haftstücke befestigt sind, die mit den Haftbereichen von an dem Körper der Frau durch hautfreundliche Klebemittel befestigte Streifen in der Weise zusammenwirken, dass die Prothese mit den auf der Haut klebenden Haltestreifen verbunden ist und von diesen wieder gelöst werden kann. Als Befestigungsmittel ist dabei beispielsweise eine Klettverbindung vorgesehen.

Brustprothesen, sofem sie nicht selbsthaftend oder in Verbindung mit Haftstreifen ausgelegt sind, werden meist in Spezialbüstenhalter mit integrierter Tasche oder auch teilweise direkt auf der Haut getragen, was durch den eingeschränkten Luftaustausch unterhalb der Prothese und schlechten Wärmetransport zu unangenehmen Wärmestaus mit Schweißbildung führen kann.

Es sind bereits auch Prothesen bekannt, die zweischichtig aufgebaut sind, und bei denen eine innere der Brust zugewandten Seite eine Kammer mit einer Flüssigkeit, vorzugsweise thixotrop eingestellt, gefüllt ist. Beim Tragen einer derartigen Brustprothese kommt es verstärkt durch die vorgenannte Hitzestaubildung und durch einen entsprechenden Schweißaustritt in dem Bereich der Flüssigkeit zu einer unerwünschten Blasenbildung. Dies geschieht vornehmlich durch die Wechselwirkung zwischen der üblicherweise aus Polyurethan bestehenden Kunststofffolie und der Flüssigkeit, welche keine feste Verbindung zu der Folie hat und der mechanischen Verformung der Folie und Flüssigkeit während des Tragens. Verstärkt wird diese Erscheinung durch die erhöhte Luftfeuchtigkeit, die sich zwischen der Brustprothese und dem Körper entwickelt. Die Blasenbildung erfolgt insbesondere bei einem Gemisch von thixotropen Flüssigkeiten mit Leichtfüllstoffen in dem dem Körper zugewandten Teil der Zweischichtprothese.

Bei Brustprothesen wurde weiterhin beobachtet, dass nahe der Prothesenoberfläche sichtbare Blasen auftreten. Insbesondere bei Silikonen, die mit Leichtfüllstoffen versehen sind, wie es in den letzten Jahren zunehmend gebräuchlich wurde, aber auch bei Prothesekammern, die mit Flüssigkeit gefüllt sind, ist eine Tendenz zur Blasenbildung verstärkt sichtbar, wenn die Prothese großen Luftdruckschwankungen z. B. durch lokale Höhenunterschiede unterworfen ist.

Eine derartige Blasenbildung ist darauf zurückzuführen, dass Silikone dazu tendieren Gase und somit auch Luft bis zu der jeweiligen Sättigungsgrenze, abhängig vom Umgebungsdruck, aufzunehmen. Dies kann mit einer Aufsaugfähigkeit eines Schwammes verglichen werden. Bei Änderung des Umgebungsdrucks versucht sich das absorbierte Gasvolumen entsprechend dem Umgebungsdruck wieder anzupassen, was bei Luftdruckverminderung zu deutlich sichtbaren Gasblasen nahe der Prothesenoberfläche führen kann. Um dieses Ausperlen des Gases in der fertigen Prothese zu verhindern werden die Silikone vor der Verarbeitung "evakuiert" um sicherzustellen, dass sich keinerlei Luft mehr im Material befindet. Dennoch ist zu beobachten, dass sich in den Brustprothesen, selbst wenn sie unmittelbar nach der Herstellung absolut luftfrei waren, also keine Blasenbildung zu beobachten war, nach einiger Zeit entsprechende Luft anreichert. Diese Luftanreicherung mit eventuell späterer Blasenbildung wird bei den üblichen Brustprothesen, bei denen die Kunststofffolien aus Polyurethanfolien bestehen, insbesondere dann beobachtet, wenn sie größere Luftdruckschwankungen, beispielsweise durch die Höhenlage des Bestimmungsortes aufgesetzt sind.

Aufgabe der Erfindung ist es, gattungsgemäße Brustprothesen derart weiterzubilden, dass eine Blasenbildung innerhalb der Brustprothese weitgehend verhindert wird.

Erfindungsgemäß wird diese Aufgabe durch die Kombination der Merkmale des Anspruchs 1 gelöst. Demnach wird zumindest ein Teil der Kunststofffolien aus gasdichtem Material hergestellt. Durch die geschaffene Gasbarriere kann an der entsprechenden Grenzfläche keine Luft bzw. kein Wasserdampf in das Silikon eindringen. Somit kann auch kein zu Blasen führender Luftaustausch bei Luftdruckänderungen auftreten.

Vorteilhafte Ausführungsformen der Erfindung ergeben sich aus den sich an den Hauptanspruch anschließenden Unteransprüchen.

Demnach kann die Brustprothese zumindest auf ihrer dem Körper zugewandten Seite eine wasserdampfdichte oder wasserdampfhemmende Kunststofffolie - als Sonderform einer gasdichten Kunststofffolie - aufweisen. Hier können also Folien mit Wasserdampfsperre eingesetzt werden. Dies ist insbesondere bei Mehrschichtprothesen von Vorteil, vorzugsweise Zweischichtprothesen, wobei hier gemäß einer Ausführungsvariante nur die körpernahe Schicht mindestens zum Teil mit einer wasserdampfdichten Kunststofffolie umgeben ist. Zumindest die mit dem Körper in Kontakt tretende Folie sollte hier wasserdampfundurchlässig sein. Gemäß einer alternativen Ausführungsform kann aber auch die ganze körpernahe Schicht bei der Zweischichtprothese mittels der wasserdampfdichten oder wasserdampfhemmenden Kunststofffolie umhüllt sein.

Eine andere gasdichte Ausführungsvariante besteht darin, dass die Brustprothese zumindest teilweise luftdicht ist, d. h. dass hier eine luftdichte Kunststofffolie eingesetzt ist.

Die gasdichten Kunststofffolien können zumindest teilweise aus einem oder mehreren der folgenden Bestandteile bestehen:

Ethylenvinylalkohol (EVOH), Polyvinylidenchlorid (PVDC), Polyvinylalkohol (PVAL), Polyamide (PA), hochverstrecktes Polypropylen (PP), Propylenoxid (HPPO), Polyethylennaphthalat (PEN).

Vorteilhaft können zu den Rohstoffen der gasdichten Kunststofffolien Nanocomposits zugegeben sein. Weiterhin können die gasdichten Kunststofffolien eine Plasmabeschichtung mit SiOx tragen oder mit Aluminium bedampft sein. Auch können die Materialien für die gasdichte Kunststofffolie mit anderen Kunststoffen, beispielsweise Polyethylen (PE), kombiniert werden.

Die Materialien für eine wasserdampfdichte Kunststofffolie können zumindest teilweise aus Kunststoffblends wie beispielsweise einem Polyurethanblend mit Polyethylen, Polypropylen oder anderen Polyolephinen und/oder Blends aus reinen Polyolephinen bestehen. Besonders vorteilhaft ist es, die wasserdampfdichte Kunststofffolie in Zweischichtprothesen einzusetzen, wo die zweite körpernahe Schicht eine thixotrope Flüssigkeit, ganz vorteilhaft eine thixotrope Flüssigkeit mit Leichtfüllstoffen, enthält.

Weitere Merkmale, Einzelheiten und Vorteile der Erfindung werden anhand eines in der Zeichnung dargestellten Ausführungsbeispiels näher erläutert.

Es zeigen:
- Fig. 1:: einen Längsschnitt durch eine Brustprothese gemäß einer ersten Ausführungsform der vorliegenden Erfindung und
- Fig. 2:: einen Längsschnitt durch eine Brustprothese gemäß einer zweiten Ausführungsform der vorliegenden Erfindung.

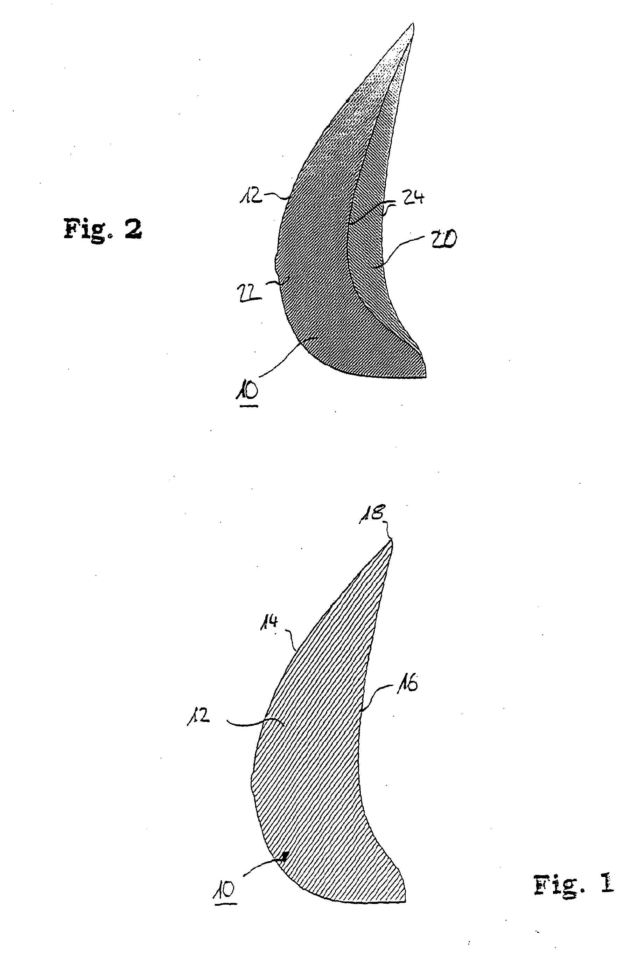

Die in Fig. 1 dargestellte Brustprothese 10 besteht aus einem schalenförmigen Körper 12 aus einer weich-elastisch eingestellten additionsvernetzenden Zweikomponenten-Silikonkautschuk-Masse, deren Außenseite durch eine Kunststofffolie 14 und deren Innenseite durch eine Kunststofffolie 16 abgedeckt ist, die längs eines gemeinsamen Umfangrandes 18 durch eine umlaufende Schweißnaht miteinander verbunden sind.

Im hier dargestellten Ausführungsbeispiel bestehen die Kunststofffolien 16 und 14 jeweils aus einem Kunststoff, der eine Gasbarriere darstellt und somit eine Sättigung des Silikons mit Luft nicht zulässt oder zumindest erheblich erschwert.

Die Ausführungsform gemäß Fig. 2 zeigt eine Brustprothese 10 als Zweischichtprothese mit einer körpernahen Schicht 20 und einer körperfernen Schicht 22. Die äußere, körperferne Schicht 22 besteht aus konventionellem additionsvernetzendem Zweikomponenten-Silikonkautschuk, also einem StandardSilikon. Sie kann alternativ auch aus einem sogenannten Light-Silikon bestehen, also einem additionsvernetzenden Zweikomponenten-Silikonkautschuk, dem eine das spezifische Gewicht der Masse emiedrigende Komponente beigemengt ist. Die innere Schicht 22 besteht aus einer thixotropen Flüssigkeit, beispielsweise einem nicht vernetzten Silikonöl, in welchem Leichtfüllstoffe eingebettet sind. Die körpernahe Schicht 20 ist in eine Kunststofffolie 24 eingeschweißt, die aus einem wasserdampfundurchlässigen Material besteht. Im hier dargestellten Ausführungsbeispiel ist die Wasserdampfsperre beispielsweise durch Polyurethanblends mit Polyethylen, Polypropylen oder anderen Polyolefinen gebildet. Demgegenüber ist die körperferne äußere Schicht 22 von einer Kunststofffolie 12 aus konventionellem Polyurethan umgeben. Die Brustprothese 10 gemäß der Ausführungsform nach Fig. 2 weist zwar nicht die Gasdichtigkeit gemäß derjenigen gemäß Fig. 1 auf, hier wird aber die Blasenbildung im Bereich der körpernahen Schicht 20 dadurch sicher verhindert oder erheblich erschwert, dass die Kunststofffolie 24 eine Wasserdampfsperre aufweist. Daher ist ein Eindringen von Wasserdampf in den Bereich der thixotropen Flüssigkeit, die mit den Leichtfüllstoffen vermischt ist, sicher verhindert.

Gemäß einer weiteren hier nicht näher dargestellten Ausführungsvariante könnte die Polyurethanschicht 12 auch durch eine Kunststofffolie ersetzt werden, die gasundurchlässig ist.

## Patentansprüche

1. Brustprothese im wesentlichen bestehend aus in Kunststoffolien eingeschweißten, der Brustform nachgebildeten Körpern aus einer Silikonkautschuk-Masse oder einem anderen weich-elastischen Material,
**dadurch gekennzeichnet,**
**dass** zumindest ein Teil der Kunststoffolien aus gasdichtem Material besteht.

2. Brustprothese nach Anspruch 1, **dadurch gekennzeichnet, dass** sie zumindest auf ihrer dem Körper zugewandten Seite eine wasserdampfdichte Kunststoffolie aufweist.

3. Brustprothese nach Anspruch 1 oder 2, **dadurch gekennzeichnet, dass** sie als Mehrschichtprothese, vorzugsweise Zweischichtprothese, ausgebildet ist, und daß nur die körpernahe Schicht mindestens zum Teil mit einer wasserdampfdichten Kunststoffolie umgeben ist.

4. Brustprothese nach einem der vorangehenden Ansprüche, **dadurch gekennzeichnet, dass** sie zumindest teilweise luftdicht ist.

5. Brustprothese nach einem der vorangehenden Ansprüche, **dadurch gekennzeichnet, dass** die gasdichte Kunststoffolie zumindest teilweise aus einem oder mehreren der folgenden Bestandteile besteht: Ethylenvinylalkohol (EVOH), Polyvinylidenchlorid (PVDC), Polyvinylalkohol (PVAL), Polyamide (PA), hochverstrecktes Polypropylen (PP), Propylenoxid (HPPO), Polyethylennaphtalat (PEN).

6. Brustprothese nach Anspruch 5, **dadurch gekennzeichnet, dass** zu den Rohstoffen der Kunststoffolien Nanocomposits oder Liquid Cristalline Polymere (LCP) zugegeben sind.

7. Brustprothese nach einem der vorangehenden Ansprüche, **dadurch gekennzeichnet, dass** die gasdichten Kunststoffolien eine Plasmabeschichtung mit SiOx tragen oder mit Aluminium bedampft sind.

8. Brustprothese nach einem der Ansprüche 5 bis 7, **dadurch gekennzeichnet, dass** die Materialen für die gasdichte Kunststoffolie mit anderen Kunststoffen, beispielsweise Polyethylen (PE), kombiniert werden.

9. Brustprothese nach einem der vorangehenden Ansprüche, **dadurch gekennzeichnet, dass** die Materialien für eine wasserdampfdichte Kunststoffolie zumindest teilweise aus Kunststoffblends wie beispielsweise einem Polyuretanblend mit Polyethylen, Polypropylen oder anderen Polyolefinen und/oder Blends aus reinen Polyolefinen bestehen.

10. Brustprothese nach einem der vorangehenden Ansprüche, **dadurch gekennzeichnet, dass** in der zweiten körpernahen Schicht einer Zweischichtprothese eine thixotrope Flüssigkeit enthalten ist.
